# EUROPEAN PATENT APPLICATION

(11) **EP 2 774 598 A1**
(43) Date of publication of application: **10.09.2014**
(21) Application number: 12844948.5
(22) Date of filing: 29.10.2012
(51) Int. Cl.: A61K 8/29, A61K 8/34, A61K 8/35, A61K 8/36, A61Q 17/04

(54) **COSMETIC**

(30) Priority: 31.10.2011 JP 2011239727
(71) Applicant: FUJI-FILM Corporation, Minato-ku Tokyo 106-8620 (JP)
(72) Inventor: KASAGI, Minako, Ashigarakami-gun Kanagawa 258-8577 (JP)
(74) Representative: Morpeth, Fraser Forrest
(86) International application number: PCT/JP2012/077912
(87) International publication number: WO 2013/065643

(57) **Abstract**

A cosmetic includes: a composite powder of titanium oxide and 4-tert-butyl-4-methoxybenzoylmethane; at least one emulsifier selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18; and an oil absorbing powder.

## Description

### Technical Field

The present invention relates to a cosmetic.

### Background Art

Various organic ultraviolet absorbers have been used to impart an ultraviolet blocking effect to cosmetics. In particular, ultraviolet shielding powders, in which inorganic powders and ultraviolet absorbers that are organic compounds are formed as a composite, are known.

For example, International Publication (WO) No. 2010/098249 discloses a composite powder in which an organic compound having an ultraviolet absorbing ability is coated on the surface of a base material powder. It is described in WO 2010/098249 that the composite powder is a composite powder in which an organic compound having an ultraviolet absorbing ability and an inorganic pigment having an ultraviolet scattering ability are formed as a composite, and is a powder type capable of being stably blended into a cosmetic, maintains a high ultraviolet absorbing effect, and is an organic ultraviolet absorber exhibiting favorable dispersibility. As this kind of composite powder, WO 2010/098249 discloses a composite powder in which a surface of fine particles of titanium oxide is coated with butyl methoxydibenzoylmethane.

Japanese Patent Application Laid-Open (JP-A) No. H07-277936 discloses an ultraviolet shielding powder containing, in predetermined amounts: an ultraviolet absorber such as 4-tert-butyl-4-methoxybenzoylmethane; wax and/or an oil gelling agent which has/have a gelling ability, such as dextrin fatty acid ester; and a powder such as titanium oxide. It is described that the ultraviolet shielding powder is an ultraviolet shielding powder in which an oily component containing an ultraviolet absorber is adsorbed to a powder together with wax and/or an oil gelling agent, and does not cause daily variation even when blended into a hydrous-system cosmetic, and thus a stable cosmetic with excellent use sensation and usability can be obtained.

JP-A No. 2011-68567 discloses a sunscreen cosmetic, in which a UVA absorber such as 4-tert-butyl-4-methoxybenzoylmethane is blended with a given spherical resin powder such as ultrafine particles of titanium oxide.

### SUMMARY OF INVENTION

Among the ultraviolet shielding powders described above, however, there are cases in which the composite powder in which the surface of fine particles of titanium oxide is coated with butyl methoxydibenzoylmethane absorbs or releases oily components in an emulsion type cosmetic when the composite powder is blended into the emulsion type cosmetic. As a result, there are cases in which changes in the viscosity of cosmetic, or deterioration in the use sensation such as with the occurrence of stickiness or a lack of appropriate spreading, occur. In addition, there are cases in which the ultraviolet absorber in the composite powder dissolves out over time or recrystallizes in the preparation. As described above, the viscosity stability or use sensation of a cosmetic is not satisfactory when a composite powder having a high ultraviolet shielding effect is blended into an emulsion type cosmetic.

An object of the invention, in view of such circumstances, is to provide a cosmetic exhibiting a high ultraviolet shielding effect, favorable viscosity stability, and a favorable use sensation.

The invention is as follows.
[1] A cosmetic, comprising:
   a composite powder of titanium oxide and 4-tert-butyl-4-methoxybenzoylmethane;
   at least one emulsifier selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18; and
   an oil absorbing powder.
[2] The cosmetic according to [1], wherein the oil absorbing powder is at least one oil absorbing powder selected from the group consisting of a porous silica powder, a crosslinked silicone powder, a porous nylon powder, a polymethyl methacrylate powder, and corn starch.
[3] The cosmetic according to [1], wherein the emulsifier is at least one selected from the group consisting of (2) a linear unsaturated alcohol having a total carbon number of from 8 to 18, (3) a branched saturated alcohol having a total carbon number of from 8 to 18, (5) a linear unsaturated fatty acid having a total carbon number of from 8 to 18, and (6) a branched saturated fatty acid having a total carbon number of from 8 to 18.
[4] The cosmetic according to [1] or [3], wherein the emulsifier is at least one selected from the group consisting of oleic acid, isostearic acid, oleyl alcohol, and isostearyl alcohol.
[5] The cosmetic according to any one of [1], [3], and [4], wherein a content of the emulsifier is from 0.0001 times to 150 times the amount on a mass basis with respect to a content of the composite powder.
[6] The cosmetic according to any one of [1] to [5], being a sunscreen cosmetic.
[7] The cosmetic according to any one of [1] to [6], wherein the composite powder comprises a surface treatment layer, including the 4-tert-butyl-4-methoxybenzoylmethane, on a surface of the titanium oxide.
[8] The cosmetic according to any one of [1] to [7], wherein an average particle diameter of the composite powder is less than 1 µm.
[9] The cosmetic according to any one of [1], [2], and [5] to [8], wherein the (1) linear saturated alcohol having a total carbon number of from 8 to 16 is at least one selected from the group consisting of capryl alcohol, decyl alcohol, lauryl alcohol, myristyl alcohol, and palmityl alcohol.
[10] The cosmetic according to any one of [1], [2], and [5] to [9], wherein the (2) linear unsaturated alcohol having a total carbon number of from 8 to 18 is at least one selected from the group consisting of octenol, decenol, dodecenol, and oleyl alcohol.
[11] The cosmetic according to any one of [1], [2], and [5] to [10], wherein the (3) branched saturated alcohol having a total carbon number of from 8 to 18 is at least one selected from the group consisting of isodecyl alcohol, isotridecyl alcohol, isotetradecyl alcohol, isopalmityl alcohol, hexyldecanol, and isostearyl alcohol.
[12] The cosmetic according to any one of [1], [2], and [5] to [11], wherein the (4) linear saturated fatty acid having a total carbon number of from 12 to 22 is at least one selected from the group consisting of lauric acid, myristic acid, palmitic acid, stearic acid, and behenic acid.
[13] The cosmetic according to any one of [1], [2], and [5] to [12], wherein the (5) linear unsaturated fatty acid having a total carbon number of from 8 to 18 is at least one selected from the group consisting of undecylenic acid, myristoleic acid, palmitoleic acid, oleic acid, elaidic acid, vaccenic acid, linoleic acid, linolenic acid, pinolenic acid, stearidonic acid, eleostearic acid, and bosseopentaenoic acid.
[14] The cosmetic according to any one of [1], [2], and [5] to [13], wherein the (6) branched saturated fatty acid having a total carbon number of from 8 to 18 is at least one selected from the group consisting of isooctanoic acid, isononanoic acid, isodecanoic acid, isoundecanoic acid, isolauric acid, isotridecanoic acid, octylic acid, isomyristic acid, isopalmitic acid, and isostearic acid.
[15] The cosmetic according to any one of [2] to [14], wherein the porous silica powder is at least one selected from the group consisting of silica, silica silylate, silica dimethyl silylate, and silica dimethicone silylate.
[16] The cosmetic according to any one of [2] to [15], wherein the crosslinked silicone powder is at least one selected from the group consisting of a silicone gel powder, a silicone rubber powder, a silicone resin powder, and a silicone elastomer.
[17] The cosmetic according to any one of [2] to [16], wherein the porous nylon powder is at least one selected from the group consisting of nylon, nylon-6, nylon-11, nylon-12, nylon-12/6/66 copolymer, nylon-6/12, and nylon-66.
[18] The cosmetic according to any one of [1] to [17], wherein the oil absorbing powder is at least one selected from the group consisting of a porous polymer formed from styrene/stearyl methacrylate/divinylbenzene or the like, a hydrogenated (styrene/isoprene) copolymer, a (butylene/ethylene/styrene) copolymer, a (styrene/acrylamide) copolymer, a (ethylene/propylene/styrene) copolymer, a clay mineral, bentonite, synthetic phlogopite, a polyamide resin, and flaky titanium oxide.
[19] The cosmetic according to any one of [1] to [18], wherein the oil absorbing powder is at least one selected from the group consisting of a (dimethicone/vinyl dimethicone) crosspolymer, a dimethicone crosspolymer, and polymethyl methacrylate (PMMA).
[20] The cosmetic according to any one of [1] to [19], wherein a volume average particle diameter of the oil absorbing powder is from 0.005 µm to 30 µm.

According to the invention, a cosmetic exhibiting a high ultraviolet shielding effect, favorable viscosity stability, and a favorable use sensation can be provided.

### DESCRIPTION OF EMBODIMENTS

The cosmetic of the invention is a cosmetic including: a composite powder of titanium oxide and 4-tert-butyl-4-methoxybenzoylmethane; at least one emulsifier selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18; and an oil absorbing powder.

The cosmetic contains a specific composite powder, a specific emulsifier, and an oil absorbing powder. Therefore, it is presumed that the dissolving out of 4-tert-butyl-4-methoxybenzoylmethane from the composite powder and recrystallization can be suppressed, and the viscosity stability and a use sensation of the cosmetic are favorable without impairing the high ultraviolet shielding performance.

The term "process" in the present specification includes not only an independent process but also a case where a process is not clearly distinguished from other processes as long as the intended purpose of the process is achieved.

In addition, the term "- (to)" in the present specification denotes a range including the numerical values described before and after "- (to)" as the minimum value and the maximum value, respectively.

Moreover, the amount of each of the components in the composition in the present specification means, in a case where a plurality of substances, which represents each of the components in the compositionb is present, the total amount of a plurality of substances present in the composition, unless otherwise noted.

Hereinafter, the invention is described.

The cosmetic according to the invention is a cosmetic including: a composite powder of titanium oxide and 4-tert-butyl-4-methoxybenzoylmethane; at least one emulsifier selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18; and an oil absorbing powder.

The cosmetic preferably has an emulsion form. The emulsion form is not particularly limited, and may be a W/O type or an O/W type.

### <Composite Powder>

The cosmetic according to the invention contains a composite powder. The composite powder is a composite powder of titanium oxide and 4-tert-butyl-4-methoxybenzoylmethane. The composite powder contains 4-tert-butyl-4-methoxybenzoylmethane, and hence has high ultraviolet shielding performance.

The composite powder is preferably a composite powder having, on the surface of titanium oxide, a surface treatment layer which contains 4-tert-butyl-4-methoxybenzoylmethane as an ultraviolet absorber. The binding mode between the surface treatment layer and titanium oxide is not particularly limited as long as 4-tert-butyl-4-methoxybenzoylmethane and titanium oxide behave integrally. The binding mode may be a chemical bond such as covalent bond or may be a nonchemical bond such as adsorption.

In addition, fatty acid and aluminum hydroxide may be contained in the surface treatment layer. The surface of the powder can be hydrophobic by inclusion of fatty acid in the surface treatment layer, and titanium oxide can be inactivated by inclusion of aluminum hydroxide.

Examples of the composite powder include the composite powders disclosed in WO 2010/098249. In addition, as the composite powder, a commercially available product can be used and examples thereof include HXMT-100ZA manufactured by TAYCA.

The content of the composite powder in the cosmetic is preferably from 0.1% by mass to 50% by mass, more preferably from 0.5% by mass to 25% by mass, and still more preferably from 1% by mass to 15% by mass, with respect to the total mass of the cosmetic. When the content is 0.1 % by mass or more, an ultraviolet blocking effect can be obtained, and when the content is 50% by mass or less, the composite powder can be stably blended in the formula.

An average primary particle diameter of titanium oxide is preferably from 1 nm to 90 nm, and more preferably from 5 nm to 50 nm, from the viewpoint of ultraviolet shielding performance and transparency of cosmetic. The average primary particle diameter of titanium oxide is the value obtained by dispersing titanium oxide, subsequently, taking the image of 1000 or more particles with a transmission electron microscope, performing image treatment of individual particles in which the images thereof have been taken, with an image analysis type particle size distribution measuring apparatus, and measuring the equivalent circle diameter. When a commercially available product is used, the average primary particle diameter of the commercially available product may be adopted as it is.

The average particle diameter of the composite powder is preferably less than 1 µm. When the average particle diameter of the composite powder is less than 1 µm, the coloring of cosmetic by the composite itself is suppressed and the so-called white powder residue also does not occur, therefore it is preferable. The average particle diameter of the composite powder is preferably from 1 nm to 500 nm and more preferably from 3 nm to 100 nm, from the viewpoints of ultraviolet shielding performance and use sensation.

The average particle diameter of the composite powder can be measured by the same method as that of measuring the average primary particle diameter.

In the case that the composite powder is blended into the cosmetic, slurry may be prepared using a dispersion medium such as silicone oil, and then the composite powder in a slurry form may be blended with other components, for example.

The content of the composite powder in the slurry is not particularly limited, and generally, is preferably from 10% by mass to 80% by mass and more preferably from 20% by mass to 60% by mass, with respect to the total mass of the slurry. When the content is 10% by mass or more, the composite powder can be stably blended into the formulation, and the content thereof is still more preferably from 20% by mass to 60% by mass.

### <Emulsifier>

The cosmetic according to the invention contains an emulsifier. The emulsifier is at least one selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18. Since the cosmetic contains a specific emulsifier, the cosmetic exhibits excellent viscosity stability and excellent use sensation.

Examples of (1) the linear saturated alcohol having a total carbon number of from 8 to 16 include capryl alcohol, decyl alcohol, lauryl alcohol, myristyl alcohol, and palmityl alcohol. When the total carbon number is less than 8, (1) the linear saturated alcohol does not contribute to the viscosity stability, and when the total carbon number is more than 16, the viscosity stability of cosmetic significantly decreases and use sensation is not also ameliorated.

A linear saturated alcohol having a total carbon number of 14, a total carbon number of 16, or the like is preferable from the viewpoint of the viscosity stability of cosmetic.

Examples of (2) the linear unsaturated alcohol having a total carbon number of from 8 to 18 include octenol, decenol, dodecenol, and oleyl alcohol. When the total carbon number is less than 8, (2) the linear unsaturated alcohol does not contribute to the viscosity stability, and when the total carbon number is more than 18, use sensation is not ameliorated.

A linear unsaturated alcohol having a total carbon number of 16, a total carbon number of 18, or the like is preferable and oleyl alcohol is still more preferable, from the viewpoint of the viscosity stability of cosmetic.

Examples of (3) the branched saturated alcohol having a total carbon number of from 8 to 18 include isodecyl alcohol, isotridecyl alcohol, isotetradecyl alcohol, isopalmityl alcohol, hexyldecanol, and isostearyl alcohol. When the total carbon number is less than 8, (3) the branched saturated alcohol does not contribute to the viscosity stability, and when the total carbon number is more than 18, use sensation is not ameliorated.

A branched saturated alcohol having a total carbon number of 16, a total carbon number of 18, or the like is preferable and isostearyl alcohol is still more preferable, from the viewpoint of the viscosity stability of cosmetic.

Examples of (4) the linear saturated fatty acid having a total carbon number of from 12 to 22 include lauric acid, myristic acid, palmitic acid, stearic acid, and behenic acid. When the total carbon number is less than 12, (4) the linear saturated fatty acid does not contribute to the viscosity stability, and when the total carbon number is more than 22, use sensation (stickiness) is not ameliorated.

A linear saturated fatty acid having a total carbon number of from 12 to 18, or the like is preferable, and palmitic acid and stearic acid are still more preferable, form the viewpoint of obtaining favorable use sensation.

Examples of (5) the linear unsaturated fatty acid having a total carbon number of from 8 to 18 include undecylenic acid, myristoleic acid, palmitoleic acid, oleic acid, elaidic acid, vaccenic acid, linoleic acid, linolenic acid, pinolenic acid, stearidonic acid, eleostearic acid, and bosseopentaenoic acid. When the total carbon number is less than 8, (5) the linear unsaturated fatty acid does not contribute to the viscosity stability, and when the total carbon number is more than 18, use sensation is not ameliorated.

A linear unsaturated fatty acid having a total carbon number of 16, a total carbon number of 18, or the like is preferable, and oleic acid is still more preferable, from the viewpoint of the viscosity stability of cosmetic.

Examples of (6) the branched saturated fatty acid having a total carbon number of from 8 to 18 include isooctanoic acid, isononanoic acid, isodecanoic acid, isoundecanoic acid, isolauric acid, isotridecanoic acid, octylic acid, isomyristic acid, isopalmitic acid, and isostearic acid. When the total carbon number is less than 8, (6) the branched saturated fatty acid does not contribute to the viscosity stability, and when the total carbon number is more than 18, use sensation is not ameliorated.

A branched saturated fatty acid having a total carbon number of 16, a total carbon number of 18, or the like is preferable, and isostearic acid is still more preferable, from the viewpoint of the viscosity stability of cosmetic.

As the emulsifier, (2) the linear unsaturated alcohol having a total carbon number of from 8 to 18, (3) the branched saturated alcohol having a total carbon number of from 8 to 18, (5) the linear unsaturated fatty acid having a total carbon number of from 8 to 18, or (6) the branched saturated fatty acid having a total carbon number of from 8 to 18 is preferable, from the viewpoints of improvement in viscosity stability and use sensation of cosmetic.

Among the alcohols described in the item (2) and (3) and the fatty acids described in the item (5) and (6), palmitic acid, stearic acid, oleic acid, isostearic acid, oleyl alcohol, or isostearyl alcohol is preferable, and oleic acid, isostearic acid, oleyl alcohol, or isostearyl alcohol is still more preferable, from the viewpoint of viscosity stability of cosmetic.

In the cosmetic according to the invention, one of the respective emulsifiers exemplified in the items (1) to (6) may be used singly, or two or more thereof may be used in combination. A mixture of the respective emulsifiers described above, such as jojoba alcohol, coconut alcohol, or coconut fatty acid may also be used.

When the respective emulsifiers described in the items (1) to (6) are used in combination, examples of a combination include oleic acid and isostearic acid, stearic acid and isostearic acid, stearic acid and oleic acid, oleyl alcohol and isostearic acid, isostearyl alcohol and isostearic acid, oleyl alcohol and stearic acid, and isostearyl alcohol and stearic acid.

The content of the alcohol described in the items (1) to (3) above is preferably from 0.01% by mass to 15% by mass, more preferably from 0.05% by mass to 10% by mass, and still more preferably from 0.1% by mass to 5% by mass, with respect to the total mass of the cosmetic, from the viewpoints of viscosity stability and use sensation. When the content is 0.01 % by mass or more, the viscosity stability tends to be improved, and when the content is 15% by mass or less, use sensation of cosmetic is improved.

The content of the fatty acid described in the items (4) to (6) above is preferably from 0.01% by mass to 15% by mass, more preferably from 0.05% by mass to 10% by mass, and still more preferably from 0.1% by mass to 5% by mass, with respect to the total mass of the cosmetic, from the viewpoints of viscosity stability and use sensation. When the content is 0.01 % by mass or more, the viscosity stability tends to be improved, and when the content is 15% by mass or less, use sensation of is improved.

The content of the emulsifier is preferably from 0.01% by mass to 15% by mass, more preferably from 0.05% by mass to 10% by mass, and still more preferably from 0.1% by mass to 5% by mass, with respect to the total mass of the cosmetic, from the viewpoints of viscosity stability and use sensation. When the content is 0.01% by mass or more, the viscosity stability tends to be improved, and when the content is 15% by mass or less, use sensation is improved.

The content of the emulsifier is preferably from 0.0001 times to 150 times, more preferably from 0.0005 times to 50 times, and still more preferably from 0.001 times to 5 times, the amount on a mass basis, with respect to the content of the composite powder. When the content is 0.0001 times or higher, the viscosity stability tends to be improved, and when the content is 150 times or lower, the dispersibility of the composite powder tends to be improved.

### <Oil Absorbing Component>

The cosmetic according to the invention contains an oil absorbing powder. Since the cosmetic contains an oil absorbing powder, the cosmetic exhibits excellent viscosity stability.

The oil absorbing powder denotes a water insoluble powder having an oil absorption in which squalane can be absorbed at 25°C in an amount of 30% by mass or more of the dead weight of powder.

As the measuring method of oil absorption, a known method may be used. The oil absorption can be measured, for example, by: placing 1 g of oil absorbing powder on a glass plate; kneading the oil absorbing powder using a spatula while dropping squalane in small steps; taking the time point at which the entire oil absorbing powder is in paste form as the end point; and taking the squalane amount (ml) per 1 g of oil absorbing powder at this time as the oil absorption.

As such an oil absorbing powder, examples thereof include at least one oil absorbing powder selected from the group consisting of a porous silica powder, a crosslinked silicone powder, a porous nylon powder, a polymethyl methacrylate powder, and corn starch.

Examples of the porous silica powder include silica, silica silylate, dimethyl silica silylate, and silica dimethicone silylate. Preferable examples thereof include silica from the viewpoint of viscosity stability. Examples of commercially available products thereof include SUNSPHERE H-31/ H-32/ H-33/ H-51/ H-52/ H-53/ H-121/ H-122/ and H-201 (manufactured by ASAHI GLASS CO., LTD.), VM-2270 Aerogel Fine Particle (manufactured by Dow Coming Toray Co., Ltd), HDK (registered trademark) H2000, HDK (registered trademark) H15, HDK (registered trademark) H18, HDK (registered trademark) H20, and HDK (registered trademark) H30 (manufactured by wacker asahikasei silicone co., ltd.), SYLOPURE (manufactured by FUJI SILYSIA CHEMICAL LTD.), TOKUSIL (manufactured by Tokuyama Corporation), and Microbead silica gel (Fuji Davison Chemical Ltd).

Examples of the crosslinked silicone powder include a silicone gel powder, a silicone rubber powder, a silicone resin powder, and a silicone elastomer, and these can be used without distinguishing from one another. As the crosslinked silicone powder, a (dimethicone/vinyl dimethicone) crosspolymer, a (dimethicone/bis-isobutyl PPG-20) crosspolymer, a dimethicone crosspolymer, a (PEG-10/lauryl dimethicone) crosspolymer, a (PEG-10 dimethicone/vinyl dimethicone) crosspolymer, a (PEG-15/lauryl dimethicone) crosspolymer, a (PEG-15/lauryl polydimethylsiloxyethyl dimethicone) crosspolymer, a (diphenyl dimethicone/vinyl diphenyl dimethicone/silsesquioxane) crosspolymer, a (dimethicone/bis-isobutyl PPG-20) crosspolymer, a (dimethicone/vinyl trimethylsiloxysilicate) crosspolymer, a (dimethicone/phenyl vinyl dimethicone) crosspolymer, a (vinyl dimethicone/lauryl dimethicone) crosspolymer, a (vinyl dimethicone/methicone silsesquioxane) crosspolymer, a (lauryl polydimethylsiloxyethyl dimethicone/bis-vinyl dimethicone) crosspolymer, and the like are preferable, and a (dimethicone/vinyl dimethicone) crosspolymer or dimethicone crosspolymer is more preferable, from the viewpoint of viscosity stability of the cosmetic.

Examples of commercially available products of the silicone gel powder include KSG-15/ 16/ 1610, KSG-18A, KSG-41, KSP-100/ 101/ 102/ 105, and KSP-300, 441/ 411, KSG-41/ 42/ 43/ 44, KSG-240/ 310/ 320/ 330/ 340/ 710/ 320Z/ 350Z (manufactured by Shin-Etsu Chemical Co., Ltd.). Examples of commercially available products of the silicone rubber powder include TREFIL E-506C, E-508, 9701 Cosmetic Powder, 9702 Powder, 9027/ 9040/ 9041/ 9045/ 9046/ 9041/ 9546 Silicone Elastomer Blend, EP-9215/ EP-9216 TI/ EP-9289 LL/ EP-9293 AL, EL-8040 ID Silicone Organic Blend (manufactured by Dow Coming Toray Co., Ltd.), Wacker-Belsil (registered trademark) RG 100 (manufactured by Wacker Asahikasei Silicone Co., Ltd.), NIKKOL SILBLEND-91 (manufactured by Nikko Chemicals Co., Ltd).

Examples of the porous nylon powder include nylon, nylon-6, nylon-11, nylon-12, a nylon-12/6/66 copolymer, nylon-6/12, and nylon-66. Nylon-6, nylon-12, and the like are preferable from the viewpoint of viscosity stability of cosmetic.

Examples of commercially available products thereof include TR-1, TR-2, and SP-500 (manufactured by TORAY INDUSTRIES INC.), POMP 605 and POMP 610 (manufactured by UBE INDUSTRIES LTD.), and NYLON POWDER (manufactured by NIKKO RICA CORPORATION).

As the polymethyl methacrylate (hereinafter, it is also simply referred to as "PMMA") powder, a methyl methacrylate crosspolymer, a (methyl methacrylate/glycol dimethacrylate) crosspolymer, a (methyl methacrylate/acrylonitrile) copolymer, a (polymethyl methacrylate/dimethyl polysiloxane graft acrylic resin) copolymer, an (ethylhexyl acrylate/methyl methacrylate) copolymer, and the like are also included, in addition to PMMA.

Examples of commercially available products thereof include TECHPOLYMER (manufactured by SEKISUI PLASTICS CO., LTD.) and MATSUMOTO MICROSPHERE (manufactured by Matsumoto Yushi-Seiyaku Co., Ltd).

As the corn starch, DRY FLO PURE (manufactured by Akzo Nobel), and the like can be used.

In addition, as the oil absorbing powder, a porous polymer formed from styrene/stearyl methacrylate/divinylbenzene or the like, a hydrogenated (styrene/isoprene) copolymer, a (butylene/ethylene/styrene) copolymer, a (styrene/acrylamide) copolymer, a (ethylene/propylene/styrene) copolymer, a clay mineral, bentonite, synthetic phlogopite, a polyamide resin, and flaky titanium oxide can also be used. Examples of commercially available products thereof include LUXELEN D (manufactured by NIHONKOKEN Co., Ltd.), TIPAQUE CR-50 (manufactured by ISHIHARA SANGYO KAISHA, LTD.), PIONEER GEL 12 PAO (manufactured by Hansen & Rosenthal KG), Jojoba Glaze HV/LV (manufactured by Nikko Chemicals Co., Ltd.), Yodosol GH41F (manufactured by Akzo Nobel) and Kunipia F/G (manufactured by KUNIMINE INDUSTRIES CO., LTD).

As the oil absorbing powder, a (dimethicone/vinyl dimethicone) crosspolymer, a dimethicone crosspolymer, and PMMA are preferable, and a (dimethicone/vinyl dimethicone) crosspolymer and PMMA are still more preferable, from the viewpoint of improving the viscosity stability and use sensation of cosmetic.

In the cosmetic according to the invention, as the oil absorbing powder, one the respective components described above may be used singly or two or more thereof may be used in combination.

The particle diameter of the oil absorbing powder is not particularly limited, and the volume average particle diameter is preferably from 0.005 µm to 30 µm, more preferably from 0.01 µm to 30 µm, and still more preferably from 0.1 µm to 30 µm, from the viewpoint of improving use sensation. When the volume average particle diameter is 0.005 µm or more, use sensation is improved without squeaky feel of skin at the time of use. In addition, when the volume average particle diameter is 30 µm or less, the surface area per unit weight of the oil absorbing powder does not become too small, decrease in oil absorption speed does not occur, and adhesion to skin is preserved. The volume average particle diameter can be measured with various commercially available particle size distribution meters or the like, and a particle size distribution meter adopting a dynamic light scattering method is used, in terms of particle size range and ease of measurement. As a commercially available measuring apparatus using dynamic light scattering, Nanotrac UPA (manufactured by NIKKISO CO., LTD.), Dynamic Light Scattering Particle Size Analyzer LB-550 (manufactured by HORIBA Ltd.), and Particle Size Analyzer for Concentrated System FPAR-1000 (manufactured by OTSUKA ELECTRONICS CO., LTD.) are exemplified.

The volume average particle diameter of the oil absorbing powder in the invention is the value measured using Particle Size Analyzer for Concentrated System FPAR-1000 (manufactured by OTSUKA ELECTRONICS CO., LTD). Specifically, the value measured according to the following manner is adopted.

That is, the method of measuring the oil absorbing powder is that the measurement is performed by diluting the oil absorbing powder with dimethicone so as to have a concentration of 1% by mass and using a quartz cell. The particle diameter can be determined as a volume average diameter, when the sample refractive index is set as 1.600, the dispersion medium refractive index is set as 1.000 (dimethicone), and the viscosity of dispersion medium is set as the viscosity of dimethicone.

The content of the oil absorbing powder is preferably from 0.01% by mass to 20% by mass, more preferably from 0.05% by mass to 15% by mass, and still more preferably from 0.1 % by mass to 10% by mass, with respect to the total mass of cosmetic. When the content of the oil absorbing powder is 0.01% by mass or more, the viscosity stability tends to be improved, and when the content of the oil absorbing powder is 20% by mass or less, the feel tends to be improved.

The content of the oil absorbing powder is preferably from 0.01 % by mass to 90% by mass, more preferably from 0.05% by mass to 50% by mass, still more preferably from 0.1% by mass to 20% by mass, and most preferably from 0.1% by mass to 10% by mass, with respect to the total mass of the composite powder, from the viewpoint of improving the viscosity stability.

### <Other Components>

### (Surfactant)

The cosmetic may contain a surfactant.

The surfactant may be any of a nonionic surfactant, an anionic surfactant, and a cationic surfactant. Examples of the ionic surfactant include an alkyl sulfonate, an alkyl sulfate, a monoalkyl phosphate, and lecithin. Examples of the nonionic surfactant include a sorbitan fatty acid ester, a glycerin fatty acid ester, an organic acid monoglyceride, a polyglycerin fatty acid ester, a propylene glycol fatty acid ester, a polyglycerin condensed ricinoleic acid ester, a sucrose fatty acid ester, a polyethylene glycol fatty acid ester, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerin fatty acid ester, a polyoxyethylene propylene glycol fatty acid ester, polyoxyethylene castor oil, polyoxyethylene cured castor oil, PEG-9 polydimethylsiloxane ethyl dimethicone, and a (dimethicone/(PEG-10/15)) crosspolymer. Among these, polyoxyethylene cured castor oil is preferable in terms of stably dispersing other oil components, which are blended in for moisture retaining property.

The content of the surfactant is preferably from 0.01% by mass to 30% by mass, more preferably from 0.05% by mass to 20% by mass, and still more preferably from 0.1% by mass to 10% by mass, with respect to the total mass of cosmetic. When the content of the surfactant is 0.01 % by mass or more, the viscosity stability tends to be improved, and when the content of the surfactant is 30% by mass or less, use sensation tends to be improved.

### (Ultraviolet Absorber other than 4-Tert-butyl-4-methoxybenzoylmethane)

The cosmetic may contain other ultraviolet absorbers besides 4-tert-butyl-4-methoxybenzoylmethane.

As the other ultraviolet absorbers, any known oil soluble and water soluble ultraviolet absorbers can be used.

Examples of the oil soluble ultraviolet absorber may include para-aminobenzoic acid, methyl para-aminobenzoate, glyceryl para-aminobenzoate, amyl para-dimethylaminobenzoate, octyl para-dimethylaminobenzoate, ethylene glycol salicylate, phenyl salicylate, octyl salicylate, butylphenyl salicylate, homomenthyl salicylate, octyl methoxycinnamate, ethoxyethyl methoxycinnamate, ethylhexyl methoxycinnamate, glyceryl monoethylhexanoate dimethoxycinnamate, hydroxymethoxybenzophenone, dihydroxydimethoxybenzophenone, butyl methoxy benzoyl methane, and octyltriazone.

Examples of the water soluble surfactant include a benzophenone-based ultraviolet absorber such as 2,4-dihydroxy benzophenone and 2,2'-dihydroxy-4-methoxy benzophenone; a benzimidazole-based ultraviolet absorber such as phenylbenzimidazole-5-sulfonic acid and a salt thereof, and phenylenebisbenzimidazole tetrasulfonic acid and a salt thereof; urocanic acid, ethyl urocanic acid ester, 2,2-(1,4-phenylene) bis-(1H-benzimidazole-4,6-disulphonic acid), and terephthalylidene dicamphor sulfonic acid.

The content of the other ultraviolet absorbers other than 4-tert-butyl-4-methoxybenzoylmethane is preferably from 0.001% by mass to 30% by mass, more preferably from 0.01 % by mass to 20% by mass, and still more preferably from 0.1 % by mass to 10% by mass, with respect to the total mass of cosmetic in terms of complementing the ultraviolet blocking performance.

The total amount of ultraviolet absorber in the cosmetic is preferably from 0.001 % by mass to 70% by mass, more preferably from 0.01% by mass to 50% by mass, and still more preferably from 0.1% by mass to 30% by mass, with respect to the total mass of cosmetic in terms of use sensation.

### (Solvent)

The cosmetic may contain a volatile oil component as a solvent. By inclusion of the volatile oil component, feel of stickiness is reduced, therefore it is preferable.

The volatile oil component means a component having a boiling point in a range of from 60°C to 260°C under normal pressure. As the volatile oil component used in the invention, a volatile silicone-based oil and a volatile hydrocarbon-based oil are exemplified.

Examples of the volatile silicone-based oil include a linear polysiloxane such as dimethylpolysiloxane (1.5 cs [1.5 × 10⁻⁶m²/s]), dimethylpolysiloxane (10 cs) (dimethicone 10 CS), methylphenyl polysiloxane, and methylhydrogen polysiloxane; a cyclic polysiloxane such as octamethylcyclotetrasiloxane, cyclopentasiloxane, decamethylcyclopentasiloxane, dodecamethylcyclohexasiloxane, and tetramethylhydrogen cyclotetrasiloxane, and caprylyl methicone. Examples of the commercially available product thereof include KF 96L-0.65, KF 96L-1, KF 96L-1.5, and KF 995 (manufactured by Shin-Etsu Chemical Co., Ltd.), SH 200-1cs, SH 200-1.5cs, SH 200-2cs, 2-1184 Fluid, SH 245 Fluid, DC 246 Fluid, DC 345 Fluid, and SS 3408 (manufactured by Dow Coming Toray Co., Ltd), and TSF 404, TSF 405, and TSF 4045 (manufactured by Momentive Performance Materials Inc).

As the volatile hydrocarbon-based oil, any of a linear volatile hydrocarbon-based oil and a branched volatile hydrocarbon-based oil may be used. Examples of such a volatile hydrocarbon-based oil include a C₈ to C₁₆ isoalkane (it is also known as isoparaffin) such as isodecane, isododecane, and isohexadecane. Examples of the commercially available product thereof include ISOPAR (registered trademark) A, ISOPAR C, ISOPAR D, ISOPAR E, ISOPAR G, ISOPAR H, ISOPAR K, ISOPAR L, and ISOPAR M (manufactured by Exxon Mobil Corporation), SHELLSOL (registered trademark) 71 (manufactured by Shell), SALTROL (registered trademark) 100, SALTROL 130, and SALTROL 220 (manufactured by Philips), ISOSOL (registered trademark) 400 (manufactured by Nippon Petrochemicals Co., Ltd.), PARLEAM (registered trademark) 4 (manufactured by NOF CORPORATION), IP SOLVENT (registered trademark) 1620 and IP Solvent 2028 (manufactured by Idemitsu Kosan Co., Ltd.), Isohexadecane and Tetraisobutane 90 (manufactured by Bayer AG), and PERMETHYL (registered trademark) 99A, PERMETHYL 101A, and PERMETHYL 102A (manufactured by Presperse LLC).

One of the volatile oil components may be used singly, or two or more thereof may be used in combination.

The content of the volatile oil component is preferably from 0.001 % by mass to 60% by mass, more preferably from 0.01% by mass to 40% by mass, and still more preferably from 0.1 % by mass to 20% by mass, with respect to the total mass of cosmetic in terms of use sensation.

### (Polyhydric Alcohol)

The cosmetic may contain a polyhydric alcohol. By inclusion of a polyhydric alcohol, use sensation (moisture retaining property) can be improved.

Examples of the polyhydric alcohol may include glycerin, 1,3-butanediol (1,3-BG), ethylene glycol, and a polysaccharide such as reduced starch syrup, sucrose, erythritol, xylitol, glucose, galactose, sorbitol, maltotriose, and trehalose. These may be used singly or two or more thereof may be used in combination.

### (Other Components)

The cosmetic may contain other components, which are commonly used for cosmetics, based on the form of emulsion or the intended use. Examples of other components may include a water soluble organic solvent such as ethanol, a chelating agent, a skin lightening agent, a moisturizer, an antioxidant, a thickener, a coloring agent, a preservative, a perfume, various oil components, and various aqueous components.

The cosmetic of the invention can be produced by appropriately blending the respective components described above and then preparing a W/O type or O/W type emulsified composition by a general method.

The form of the cosmetic is not particularly limited, and examples thereof include skin care cosmetics such as skin toner (lotion), milky lotion, cream, eye cream, serum, massage materials, pack materials, ointment, cream, and body cream, and makeup cosmetics such as makeup base.

It is particularly preferable that the cosmetic of the invention is used as a sunscreen cosmetic due to high ultraviolet shielding performance and favorable use sensation.

### EXAMPLES

Hereinafter, the invention is described in detail with reference to Examples. However, the invention is not limited thereto.

### [Example 1]

After sufficiently dispersing (1) cyclopentasiloxane, (2) HXMT-100ZA (manufactured by TAYCA) and (3) dimethicone 10 CS at room temperature using a disperser so as to have the final concentration (% by mass) shown in Table 1 below, (4) PEG-9 polydimethylsiloxane ethyl dimethicone (manufactured by Shin-Etsu Chemical Co., Ltd.), (5) oleic acid as an emulsifier (LUNAC O-V (manufactured by Kao Corporation)), (6) a (dimethicone/vinyl dimethicone) crosspolymer (KSG-16 having a volume average particle diameter of 5 µm, and manufactured by Shin-Etsu Chemical Co., Ltd.) as an oil absorbing powder, and (7) a (dimethicone/(PEG-10/15) crosspolymer (KSG-210 manufactured by Shin-Etsu Chemical Co., Ltd.) were mixed by agitation to perform homogenization, thereby obtaining the oil phase component. Subsequently, a predetermined amount of (8) ethanol, (9) 1,3-butylene glycol (1,3-BG) and (11) water (a portion thereof) were weighed, mixed by agitation at room temperature and homogenized, thereby obtaining the aqueous phase component. Emulsification was performed by gradually adding the aqueous phase component to the oil phase component while agitating the oil phase component with a homomixer, (10) phenoxyethanol was added thereto, and the homogenization was performed by adding the rest of the water, thereby obtaining emulsion 1 containing the respective components at the blending amounts shown in Table 1 below.

### [Example 2 to Example 16, and Comparative Example 1 to Comparative Example 7]

Emulsions 2 to 16 according to Examples 2 to 16 and emulsions 17 to 23 according to Comparative Examples 1 to 7 were obtained in the same manner as in Example 1 except that the kind or amount (% by mass) of (5) the emulsifier and the kind or amount (% by mass) of (6) the oil absorbing powder described in Example 1 were changed to those shown in Table 1. In a case where the fatty acid or alcohol selected was solid at room temperature, the fatty acid or the alcohol was dissolved by heating in advance, and then mixed into the dispersion of (1) to (3), and (4), (6), and (7), at 80°C, emulsification was performed by gradually adding the aqueous phase component that was heated at 80°C, subsequently, (10) was added to the emulsion, and homogenization was performed by adding the rest of the water, thereby obtaining the emulsion containing the respective components at the blending amounts shown in Table 1 below.

PMMA manufactured by Matsumoto Yushi-Seiyaku Co., Ltd. was used as the oil absorbing powder, and calcium carbonate manufactured by SAKAI CHEMICAL INDUSTRY CO., LTD. was used as the non-oil absorbing powder. The volume average particle diameter of each of the oil absorbing powders used was 5 µm.

### <Evaluation>

### (1) Evaluation of Viscosity Stability

The viscosity (initial viscosity) of the emulsions 1 to 23 was measured with a BL-type viscometer (manufactured by TOKI SANGYO CO., LTD.) at room temperature (25°C) after the emulsions 1 to 23 were left to stand for one day. Thereafter, the viscosity (viscosity over time) at 25°C was measured using a BL-type viscometer after two days in a high temperature bath at 40°C.

The rate of change of viscosity with respect to the initial value was defined as the index for evaluating the stabilization, which indicates the extent to which the increase or decrease in viscosity from the initial value was suppressed, by the following Expression. The results are shown in Table 1.

Rate of change of viscosity with respect to initial value (%) = [(viscosity over time - initial viscosity)/(initial viscosity)] × 100

The viscosity stability was evaluated as (A) "excellent" in a case where the rate of change of viscosity was ±30% or less. The viscosity stability was evaluated as (C) "inappropriate" in a case where the rate of change of viscosity was more than ±30%, or separation of the aqueous phase and oil phase occurred. The results are shown in Table 1.

### (2) Evaluation of Use Sensation

Each of the emulsions obtained as described above was applied to the face by ten expert panelists, and the evaluation was performed with regard to the two criteria of spreading at the time of application and stickiness immediately after application, in five grades from outstanding (5 points) to extremely poor (1 point). The average of the evaluation results by the ten expert panelists was determined, and then the evaluation was performed such that an average of from 1 to less than 2 was ranked as C, an average of from 2 to less than 3 was ranked as B, an average of from 3 to less than 4 was ranked as A, and an average of from 4 to 5 was ranked as S. The results are shown in Table 1.

### (3) Overall Determination

Each of the emulsions was evaluated as (S) excellent as a commodity, (A) favorable as a commodity, (B) possible as a commodity, or (C) inappropriate as a commodity, based on the results of (1) and (2) described above. The results are shown in Table 1.

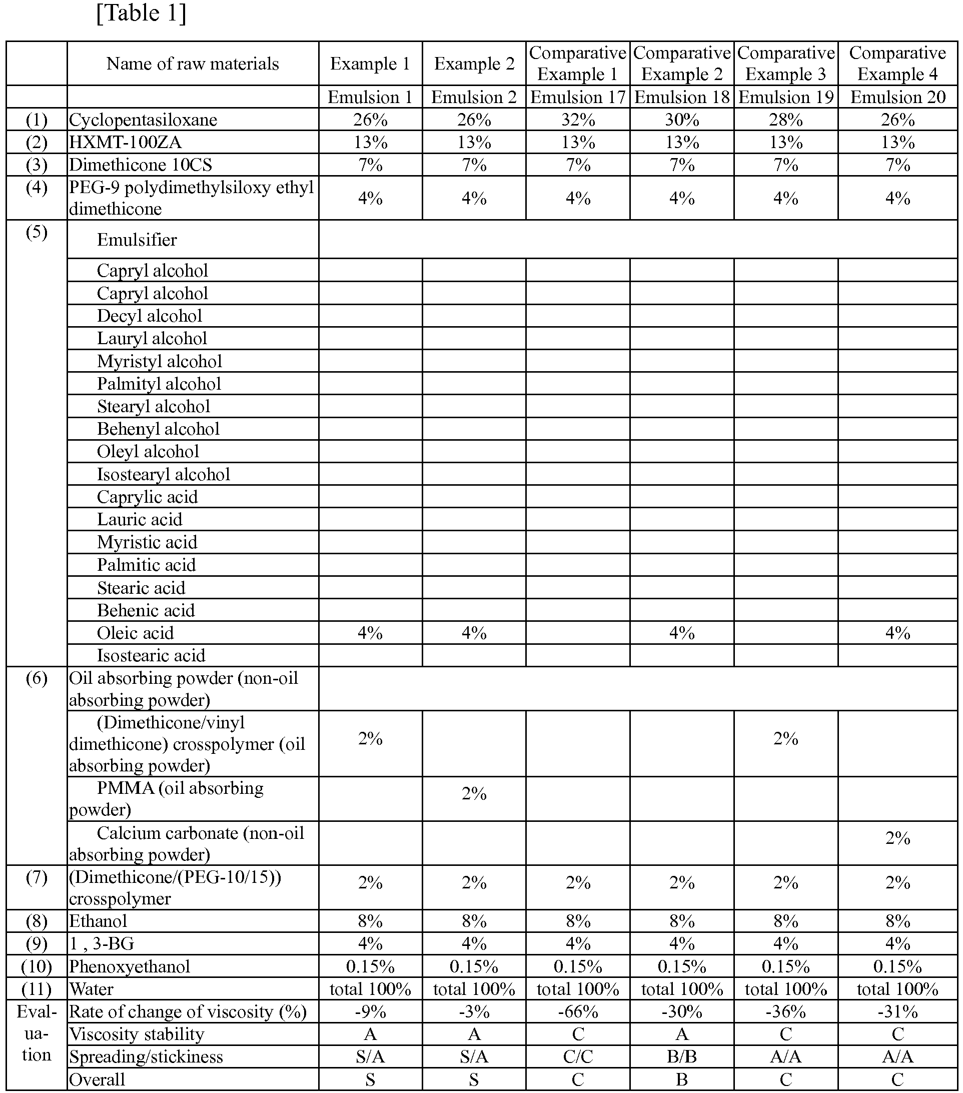

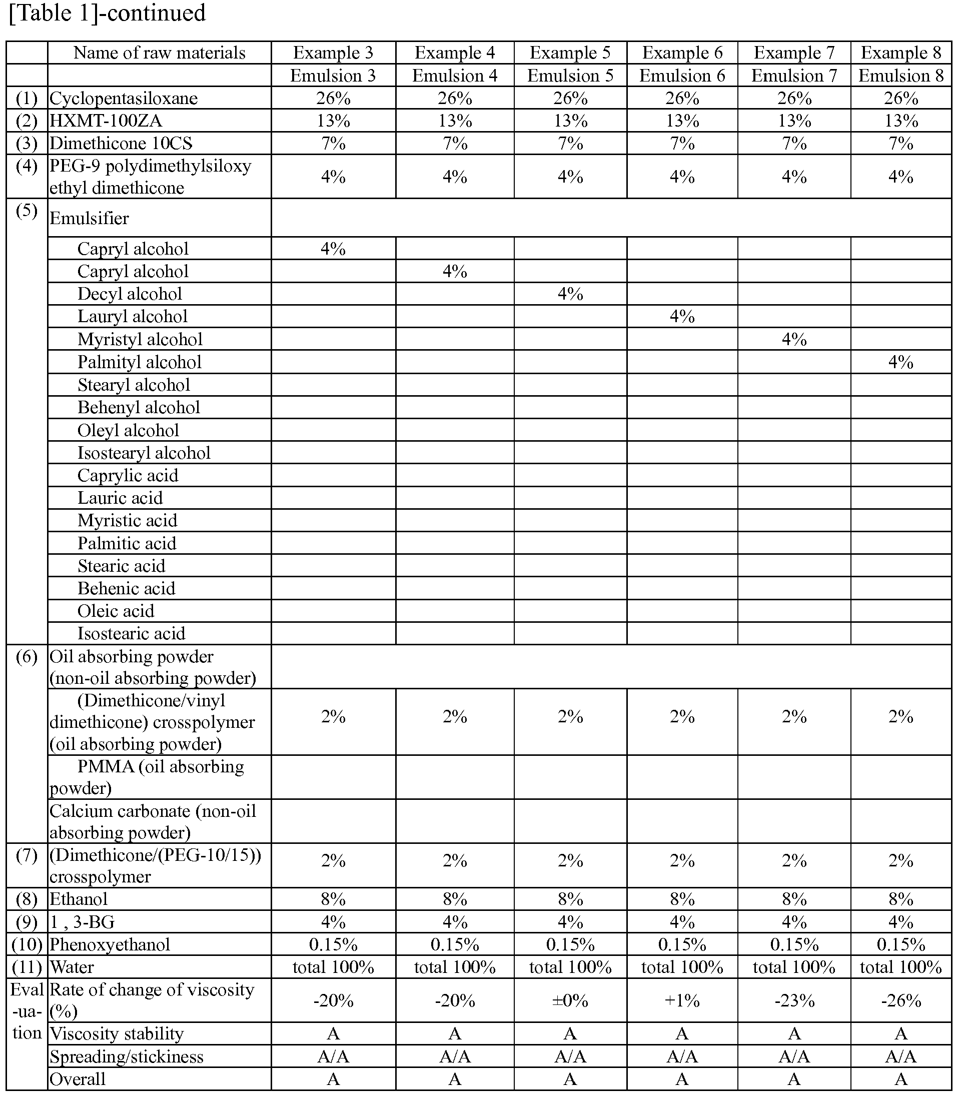

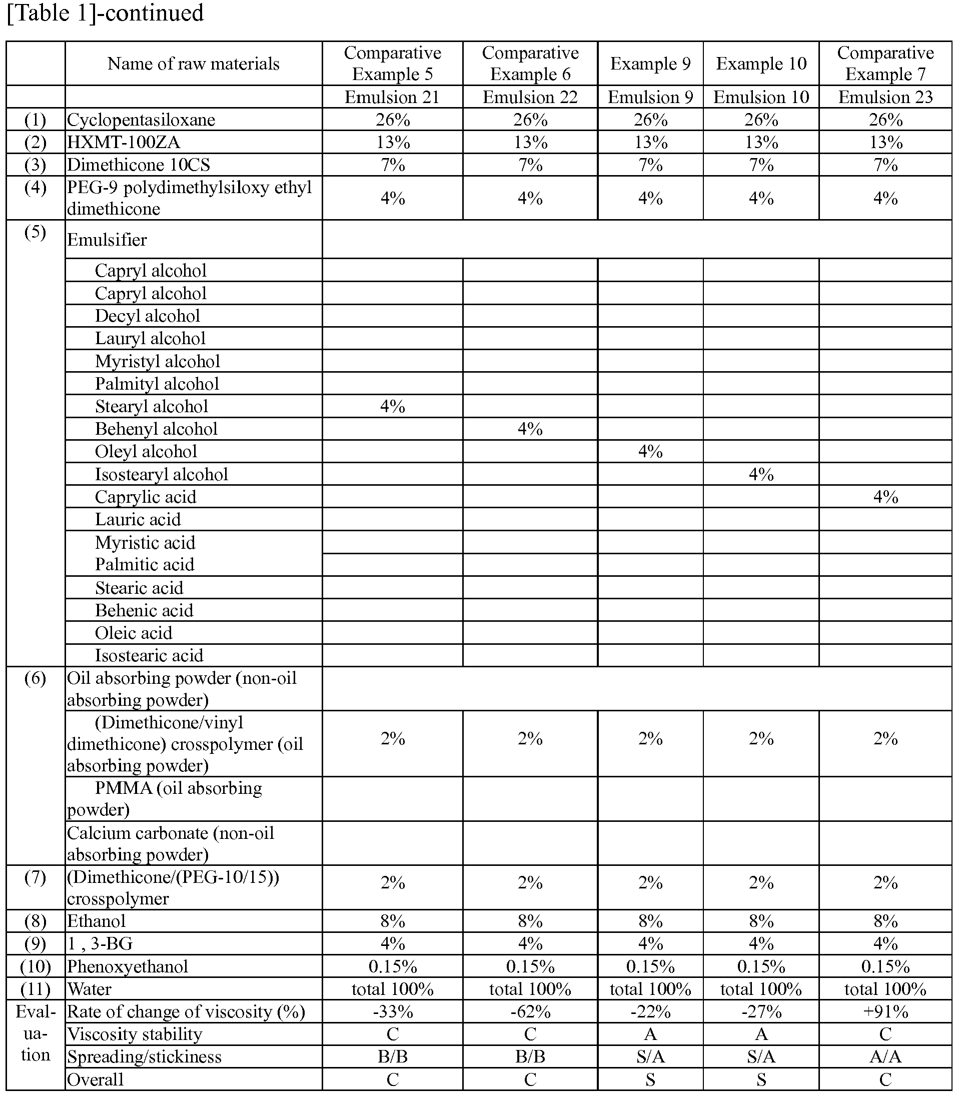

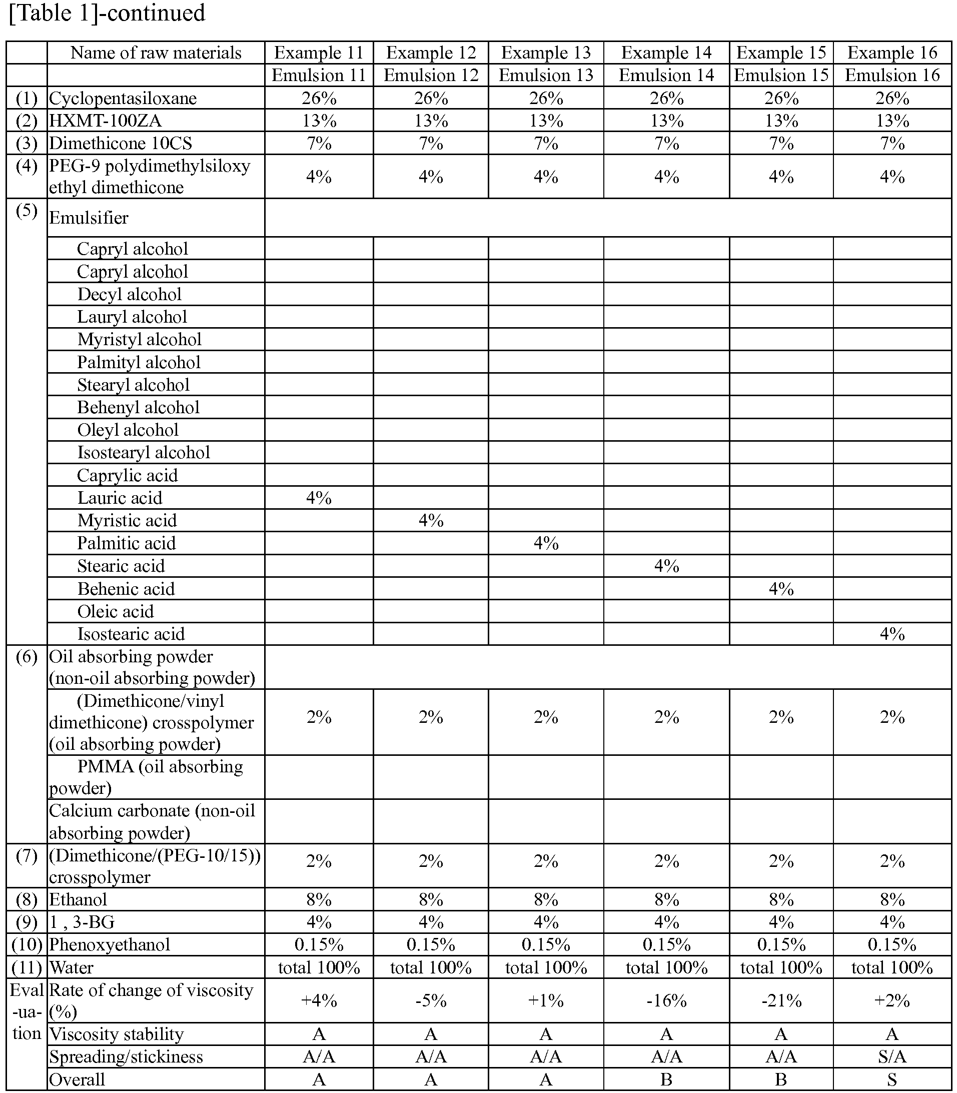

As shown in Table 1, all of the emulsions 1 to 16 according to Examples 1 to 16 exhibited a small rate of change of viscosity and excellent viscosity stability, compared with the emulsions 17 to 23 according to Comparative Examples 1 to 7. In addition, the emulsions 1 to 16 according to Examples 1 to 16 also exhibited a favorable result in the evaluation of use sensation with respect to spreading and stickiness.

In particular, the evaluation of use sensation was particularly high for Examples 1, 2, 9, 10, and 16, in which a linear unsaturated alcohol, a branched saturated alcohol, a linear unsaturated fatty acid, or a branched saturated fatty acid was used as the emulsifier.

In contrast, the evaluation of use sensation with respect to spreading and stickiness was poor and the viscosity stability was significantly impaired in Comparative Examples 1 to 4, in which an oil absorbing powder was not used, and in Comparative Examples 5 to 7, in which the specific emulsifier according to the invention was not used.

### [Example 15 and Example 16]

A W/O type emulsified UV milk (Table 2) and an O/W type emulsified UV milk (Table 3) are formulated according to the formulae shown in Table 2 and Table 3, respectively. The numerical values in Table 2 and Table 3 mean % by mass. HXMT-100ZA (manufactured by TAYCA) was used as the composite powder of "1. titanium oxide and t-butyl methoxydibenzoylmethane" in Table 2 and Table 3. The volume average particle diameter of each of the oil absorbing powders used was 5 µm.

**[Table 2]**

| W/O emulsified UV milk | | |
|---|---|---|
| 1 | Titanium oxide | 15% |
| | t-Butyl methoxydibenzoylmethane | |
| 2 | Cyclopentasiloxane | 17% |
| 3 | Dimethicone | 10% |
| 4 | PEG-9 polydimethylsiloxy ethyl dimethicone | 4% |
| 5 | Diphenylsiloxy phenyl trimethicone | 3% |
| 6 | Ethanol | 3% |
| 7 | 1,3-BG | 3% |
| 8 | Sorbitan sesquioleate | 1% |
| 9 | Isostearic acid | 0.50% |
| 10 | (Dimethicone/(PEG-10/15)) crosspolymer | 0.50% |
| 11 | (Dimethicone/vinyl dimethicone) crosspolymer | 0.50% |
| 12 | (Methyl methacrylate/glycol dimethacrylate) crosspolymer | 0.50% |
| 13 | Phenoxy ethanol | 0.30% |
| 14 | Water | total 100% |

**[Table 3]**

| O/W emulsified UV milk | | |
|---|---|---|
| 1 | Titanium oxide | 9% |
| | t-Butyl methoxydibenzoylmethane | |
| 2 | Isotridecyl isononanoate | 5% |
| 3 | Ethanol | 5% |
| 4 | Octyldodecyl myristate | 4% |
| 5 | 1 , 3-BG | 4% |
| 6 | Ethylhexyl methoxycinnamate | 4% |
| 7 | Cyclopentasiloxane | 4% |
| 8 | (Butyl acrylate/glycol dimethacrylate) crosspolymer | 1% |
| 9 | PEG-60 hydrogenated castor oil | 1% |
| 10 | Squalane | 1% |
| 11 | PMMA | 1% |
| 12 | Isostearic acid | 0.50% |
| 13 | Phenoxy ethanol | 0.30% |
| 14 | Water | total 100% |

Both of the W/O type emulsified UV milk (Table 2) and the O/W type emulsified UV milk (Table 3) are cosmetics that exhibiting a high ultraviolet shielding effect, excellent viscosity stability, and an excellent use sensation since both of the W/O type emulsified UV milk (Table 2) and the O/W type emulsified UV milk (Table 3) are cosmetics containing the composite powder, emulsifier, and oil absorbing powder according to the invention.

As described above, according to the invention, a cosmetic exhibiting a high ultraviolet shielding effect, excellent viscosity stability, and an excellent use sensation can be provided.
entirety of the disclosure of Japanese Patent Application No. 2011-239727 is incorporated herein by reference. All documents, patent applications, and technical standards described herein are incorporated by reference to the same extent as a case in which each of the documents, patent applications, and technical standards is individually and specifically incorporated by reference herein. The foregoing description with regard to the exemplary embodiments of the invention is for the purpose of illustration and explanation, and is not intended to be exhaustive or limit the invention to the precise forms disclosed. Although it is clear, it is appreciated by those skilled in the art that many modifications or changes may be made in these embodiments without departing from the principles and spirit of the invention. The embodiments described above are selected in order to best explain the principles of the invention and practical applications, to provide various embodiments applicable to particular uses estimated or various modifications, and to aid those skilled in other arts in understanding the invention. It is intended that the scope of the invention is defined in the appended claims and their equivalents.

## Claims

1. A cosmetic, comprising:
a composite powder of titanium oxide and 4-tert-butyl-4-methoxybenzoylmethane;
at least one emulsifier selected from the group consisting of (1) a linear saturated alcohol having a total carbon number of from 8 to 16, (2) a linear unsaturated alcohol having a total carbon number of from 8 to 18, (3) a branched saturated alcohol having a total carbon number of from 8 to 18, (4) a linear saturated fatty acid having a total carbon number of from 12 to 22, (5) a linear unsaturated fatty acid having a total carbon number of from 8 to 18, and (6) a branched saturated fatty acid having a total carbon number of from 8 to 18; and
an oil absorbing powder.

2. The cosmetic according to claim 1, wherein the oil absorbing powder is at least one oil absorbing powder selected from the group consisting of a porous silica powder, a crosslinked silicone powder, a porous nylon powder, a polymethyl methacrylate powder, and corn starch.

3. The cosmetic according to claim 1 or 2, wherein the emulsifier is at least one selected from the group consisting of (2) a linear unsaturated alcohol having a total carbon number of from 8 to 18, (3) a branched saturated alcohol having from 8 to 18, (5) a linear unsaturated fatty acid having a total carbon number of from 8 to 18, and (6) a branched saturated fatty acid having a total carbon number of from 8 to 18.

4. The cosmetic according to any one of claims 1 to 3, wherein the emulsifier is at least one selected from the group consisting of oleic acid, isostearic acid, oleyl alcohol, and isostearyl alcohol.

5. The cosmetic according to any one of claims 1 to 4, wherein a content of the emulsifier is from 0.0001 times to 150 times the amount on a mass basis with respect to a content of the composite powder.

6. The cosmetic according to any one of claims 1 to 5, being a sunscreen cosmetic.

7. The cosmetic according to any one of claims 1 to 6, wherein the composite powder comprises a surface treatment layer, including the 4-tert-butyl-4-methoxybenzoylmethane, on a surface of the titanium oxide.

8. The cosmetic according to any one of claims 1 to 7, wherein an average particle diameter of the composite powder is less than 1 µm.

9. The cosmetic according to any one of claims 1 to 8, wherein a volume average particle diameter of the oil absorbing powder is from 0.005 µm to 30 µm.
